Europäisches Patentamt

(19) European Patent Office  (11) Publication number: **0 221 032**

Office européen des brevets  **B1**

(12) **EUROPEAN PATENT SPECIFICATION**

(45) Date of publication of patent specification: **02.01.91**  (51) Int. Cl.⁵: **C 07 D 491/04,** A 61 K 31/40
// (C07D491/04, 311:00,
209:00)

(21) Application number: **86830304.1**

(22) Date of filing: **23.10.86**

(54) **Sodium salt of etodolic acid and process for its preparation.**

(30) Priority: **25.10.85 IT 2262585**

(43) Date of publication of application:
**06.05.87 Bulletin 87/19**

(45) Publication of the grant of the patent:
**02.01.91 Bulletin 91/01**

(84) Designated Contracting States:
**AT BE CH DE ES FR GB LI LU NL SE**

(56) References cited:
**US-A-4 076 831      US-A-4 520 203
US-A-4 501 899      US-A-4 544 757
US-A-4 515 961**

**JOURNAL OF MEDICINAL CHEMISTRY, vol. 19,
no. 3, 1976, pages 391-395, American Chemical
Society, Washington, D.C., US; C.A. Demerson
et al.: "Etodolic acid and related compounds.
Chemistry and antiinflammatory actions of
some potent Di- and trisubstituted**

**The file contains technical information
submitted after the application was filed and
not included in this specification**

(73) Proprietor: **FARMAKA S.r.l.
Via Vetreria, 1
Grandate (Como) (IT)**

(72) Inventor: **Casero, Riccardo
Via Rovascino, 21
Lipomo Como (IT)**

(74) Representative: **Aimi, Luciano et al
c/o Società Italiana Brevetti S.p.A. Via Carducci
8
I-20123 Milano (IT)**

Courier Press, Leamington Spa, England.

# EP 0 221 032 B1

**Description**

The present invention concerns the preparation of salts of 1,8-diethyl-1,3,4,9-tetrahydropyran-[3,4-b]-indol-1-acetic acid, hereinafter briefly called etodolic acid, and particularly the preparation of the water-soluble sodium salt.

In U.S. Patent No. 3,843,681 the preparation of indolic derivatives is described, which are characterized by the presence of a 1,3,4,9-tetrahydropyran (or thiopyran) [3,4-b]-indole group substituted in position 1, and possibly also other positions. Such derivatives are described as anti-inflammatory, analgesic, antibacterial and antifungal agents. The use of etodolic acid as an anti-inflammatory agent is described in Demerson et al., Jour. Med. Chem. 19(3) (1976) 391—395. Such indolic derivatives, among which also etodolic acid is included, show, however, the disadvantage of generally being as such water-insoluble, and this restricts their possible ways of administration only to the oral route, with a consequent possibility of gastro-enteric intolerance, as it is generally reported with regard to most of the anti-inflammatory agents administered by mouth.

In order to obtain derivatives of said compounds, which could possibly the water-soluble, a research has been carried out, the results of which form the object of this invention.

Etodolic acid was taken into consideration as a typical representative, particularly appreciated from the pharmacological point of view, of said derivatives and some typical salts thereof were prepared. While also most of the so obtained salts were practically fully water-insoluble, at least from the practical point of view, it was found that sodium salt, particularly if prepared under suitable conditions, is, on the contrary, easily water-soluble with the formation of solutions sufficiently stable during time also at temperatures higher than normal room-temperatures.

In order to avail oneself of a derivative usable in therapy, it is necessary, in fact, to obtain a highly soluble substance, also considering the doses of etodolic acid which have to be administered for getting an efficient therapeutic action (at least 200 mg twice a day in the adult man). By employing as bases for salification some amino acids such as arginine and lysine or potassium, which are often utilized in substances having anti-inflammatory and analgesic action for injectable preparations, the respective salts of etodolic acid were prepared, which, however, turned out to be almost completely water-insoluble in the case of amino acids and where characterized by a very poor solubility (2%) in the case of potassium salt. In this latter case the obtained solutions are deeply coloured and not much stable, when submitted to quickened ageing tests.

The sodium salt of etodolic acid, which appears in the form of white crystal powder, turned out on the contrary highly water-soluble (>20% by weight) thus producing solutions, which are only feebly yellow-coloured and, above all, are very stable during time.

The salification of etodolic acid can be easily carried out, for instance, by dissolving the acid into ethanol and slowly adding an aqueous solution of sodium hydroxide or carbonate. The resulting white crystalline product provides, however, deeply brown-coloured aqueous solutions, probably owing to the presence of by-products or impurities.

By working, on the contrary, in an anhydrous medium, as described hereinafter, a white, very water-soluble powder is obtained, which provides weakly yellow-coloured stable solutions.

The present invention will now be more particularly described on the basis of an embodiment which in no case must be regarded as limiting the invention.

## Example 1

Sodium salt of etodolic acid

28.73 g of etodolic acid are dissolved into 100 ml of anhydrous ethanol and to the obtained solution, kept under stirring, a solution consisting of 2.3 g metallic sodium dissolved into 100 ml of anhydrous ethanol is slowly added by instalments. At the end of the addition, the stirring is continued for 15 min. and then the solution is concentrated to dryness under vacuum in a rotating evaporator.

The obtained residue, after addition of 200 ml petroleum ether, is separated by filtration and washed firstly with petroleum ether, then with diethyl ether.

After a new desiccation, the product is finely powdered and suspended into 200 ml petroleum ether, by stirring for 15 min., then filtered and washed with diethyl ether. After a final desiccation, 30 g of sodium salt are obtained in the form of white micro-crystalline powder, easily soluble in water.

The 20% solution has a pH of 8.7, while a 7.3% solution has a pH of 7.8.

Elemental analysis for $C_{17}H_{20}NO_3Na$ (M.W. 309.34):
Calculated: C 66.9%; H 6.51%; N 4.53%
Found: C 66.33%; H 6.58%; N 4.37%

A thin layer chromatography through HPTLLC process of 10 mg of the so obtained sodium salt on silica gel (60 F Silicagel), using toluene-ethanol (70:30) as the development solvent, shows at the ultra-violet light of 254 n, only on spot with Rf = 0.60.

The infra-red absorption spectrum shows typical maxima at the following wavelengths: between 3,500 and 3,300 $cm^{-1}$ for the secondary amino group, between 1,080 and 1,040 $cm^{-1}$ for the cyclic ether, at 1,590

2

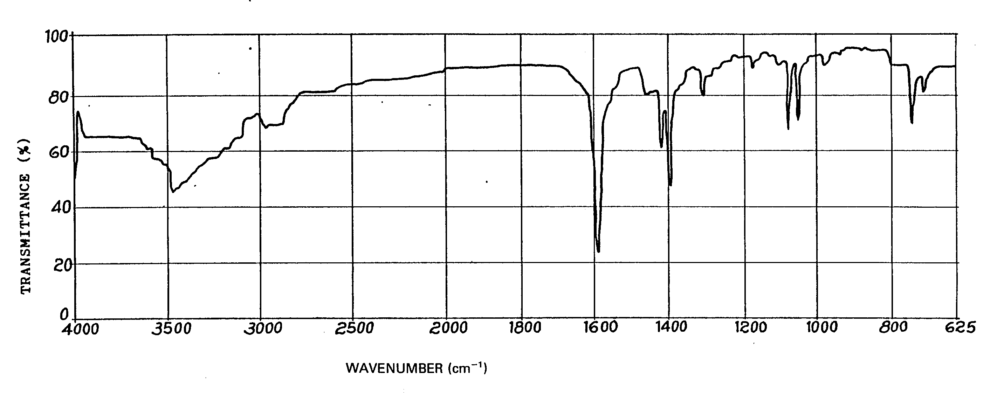

cm$^{-1}$ for the salified carboxyl and at 740 cm$^{-1}$ for the three adjacent hydrogens at positions 5, 6 and 7. The obtained spectrum is reproduced in the enclosed figure.

For the identification reaction, 0.1 g of sodium salt are dissolved into 5 ml water and the solution is acidified by means of diluted hydrochloric acid, thus obtaining a precipitate which is filtered and thoroughly washed with water. After desiccation, the precipitate shows a melting point of 147—148°C, characteristic of the free etodolic acid.

The following tables No. 1, 2 and 3 respectively show the stability data of sodium etodolate in aqueous solutions at 10% and 20% at various temperatures and again at 10% after sterilization in autoclave in a closed container.

## TABLE 1

Stability of sodium salt of etodolic acid in a 10% aqueous solution.

| | At starting point | 45°C | | | 25°C | | | 5°C | | |
|---|---|---|---|---|---|---|---|---|---|---|
| | | 1 month | 2 month | 3 month | 1 month | 2 month | 3 month | 1 month | 2 month | 3 month |
| Titre % (HPLC) | 100 | 100 | 99 | 100 | 100 | 100 | 99 | 99 | 100 | 100 |
| pH | 7.8 | 7.8 | 7.8 | 7.7 | 7.8 | 7.85 | 7.8 | 7.8 | 7.8 | 7.75 |
| Colour | pale yellow | unchanged | unchanged | unchanged | unchanged | unchanged | unchanged | unchanged | unchanged | unchanged |

EP 0 221 032 B1

## TABLE 2

Stability of sodium salt of etodolic acid in a 20% aqueous solution.

| | At starting point | 45°C | | | 25°C | | | 5°C | | |
|---|---|---|---|---|---|---|---|---|---|---|
| | | 1 month | 2 month | 3 month | 1 month | 2 month | 3 month | 1 month | 2 month | 3 month |
| Titre % (HPLC) | 100 | 99 | 98 | 98 | 100 | 99 | 100 | 99 | 100 | 97 |
| pH | 8.7 | 8.6 | 8.7 | 8.7 | 8.7 | 8.6 | 8.7 | 8.7 | 8.7 | 8.6 |
| Colour | pale yellow | unchanged | deep yellow | deep yellow | unchanged | unchanged | unchanged | unchanged | unchanged | unchanged |

EP 0 221 032 B1

TABLE 3
Stability of sodium salt of etodolic acid after sterilization
at 120°C in autoclave for 20 min.

| | At starting-point | After sterilization |
|---|---|---|
| Titre (%) (HPLC) | 100 | 100 |
| pH | 7.8 | 7.8 |
| Appearance of the solution | limpid | limpid |
| Colour of the solution | pale yellow | pale yellow |

As it may be noticed from the reported data, the sodium etodolate appears quite stable under the most different storage and treatment conditions.

The so obtained sodium etodolate, thanks to this solubility, which can even exceed 20% by weight at normal temperature, is suitable for the preparation of various pharmaceutical formulations both for oral and particularly parenteral and rectal use. Tablets, pills, granules, syrups and solutions for oral administration can thus be prepared, as well as sterile aqueous solutions for injections or for hypodermoclysis and also ovules or suppositories.

The following non-limiting example explains the preparation of suppositories based on sodium etodolate.

Example 2

69.3 g of an excipient for suppositories, known by the name of Suppocire AM, are heated to a temperature between 45° and 50°C into a container provided with stirrer. After complete melting 12 g of sodium etodolate are slowly added, always under stirring. At the end of the addition the mass is maintained under stirring and, when the temperature has dropped to 40°C, it is poured into the suppository mould, thus obtained suppositories each weighing 2.71 g on average and containing 0.40 g of sodium etodolate and 2.31 g of excipient.

Having thus described a preferred embodiment of the present invention, changes and/or alterations can obviously be made without departing for this reason from the scope of the invention.

**Claims**

1. A process for preparing sodium salt of 1,8-diethyl-1,3,4,9-tetrahydropyran-[3,4-b]indol-1-acetic acid (etodolic acid), characterized in that said acid is reacted under stirring in an anhydrous solvent with sodium alcoholate and, after evaporation, the residue is washed twice with petroleum ether and desiccated.

2. The process according to claim 1, characterized in that said anhydrous solvent is anhydrous ethanol and said sodium alcoholate is sodium ethoxide.

3. The sodium salt of etodolic acid prepared according to claim 1 or 2, characterized in that said salt provides aqueous solutions which are feebly yellow-coloured at concentration up to at least 20% by weight.

4. Pharmaceutical preparations having anti-inflammatory, analgesic and antipyretic action, characterized in that they contain an effective quantity of sodium etodolate.

**Patentansprüche**

1. Verfahren zur Herstellung des Natriumsalzes von 1,8-Diäthyl-1,3,4,9-tetrahydropyran-[3,4-b]indol-1-essigsäure (Etodolsäure), dadurch gekennzeichnet, daß die Säure unter Rühren in einem wasserfreien Lösungsmittel mit Natriumalkoholat umgesetzt wird und der Rückstand nach dem Eindampfen zweimal mit Petroläther gewaschen und getrocknet wird.

2. Verfahren nach Anspruch 1, dadurch gekennzeichnet, daß das wasssserfreie Lösungsmittel wasserfreies Äthanol und das Natriumalkoholat Natriumäthoxid ist.

3. Das Natriumsalz von Etodolsäure, hergestellt gemäß Anspruch 1 oder 2, dadurch gekennzeichnet, daß das Salz wässerige Lösungen ergibt, die in Konzentrationen bis mindestens 20 Gew.-% schwach gelb gefärbt sind.

4. Pharmazeutische Präparationen mit entzündungshemmender, analgetischer und antipyretischer Wirkung, dadurch gekennzeichnet, daß sie eine wirksame Menge an Natriumetodolat enthalten.

# EP 0 221 032 B1

## Revendications

1. Procédé de préparation du sel de sodium de l'acide, 1,8-diéthyl-1,3,4,9-tétrahydropyran-[3,4-b]indol-1-acétique (acide étodolique), caractérisé en ce qu'on fait réagir cet acide en l'agitant dans un solvant anhydre avec un alcoolate de sodium et, après, évaporation, on lave le résidu deux fois avec de l'éther de pétrole, et on le déshydrate.

2. Procédé selon la revendication 1, caractérisé en ce que le solvant anhydre, est l'éthanol anhydre, et en ce que l'alcoolate de sodium est l'éthylate de sodium.

3. Sel de sodium de l'acide étodolique, préparé selon la revendication 1 ou 2, caractérisé en ce que ce sel forme des solutions aqueuses légèrement colorées en jaune à des concentrations allant jusqu'à au moins 20% en poids.

4. Compositions pharmaceutiques ayant une activité anti-inflammatoire, analgésique et antipyrétique, caractérisées en ce qu'elles contiennent une quantité efficace d'étodolate de sodium.

WAVENUMBER (cm⁻¹)

EP 0 221 032 B1